# EUROPEAN PATENT APPLICATION

(11) **EP 0 532 359 A1**
(43) Date of publication of application: **17.03.1993**
(21) Application number: 92308314.1
(22) Date of filing: 11.09.1992
(51) Int. Cl.: G01N 33/577, G01N 33/58, C12N 15/02, C12M 3/00

(54) **Method of distinguishing cells**

(30) Priority: 13.09.1991 JP 262953/91
(71) Applicant: SHIMADZU CORPORATION, Nakagyo-ku, Kyoto 604 (JP)
(72) Inventor: Takano, Jun, Manhaimu-Gojo 922, Kyoto, Kyoto 615 (JP); Jikuya, Hiroyuki, Nishikyogokudaimon-haitsu 405, Kyoto Kyoto 617 (JP); Aoyama, Yoshihiro, Shimadzu-Syuneiryo, 18, Kyoto, Kyoto 615 (JP); Abe, Hirohisa, Imadeagawa-Horikawa-Jutaku 711, Kyoto, Kyoto 602 (JP); Okada, Manami, Nishigyo-ku, Kyoto, Kyoto 610-11 (JP)
(74) Representative: Perry, Robert Edward

(57) **Abstract**

Hybridoma cells are cultured in a microencapsulated state, whereby the antibodies produced by certain hybridoma cells are retained in the capsules. An antibody-binding substance labeled with a fluorescent substance, for instance, is reacted with the antibodies in the capsules to thereby detect the presence or absence of an antibody in each capsule and thus distinguish antibody-producing hybridomas from cells incapable of antibody production. Antibody-producing hybridoma cells can be distinguished from cells incapable of antibody production by a novel method other than ELISA.

## Description

### FIELD OF THE INVENTION

The present invention relates to a method of distinguishing antibody-producing hybridomas from cells incapable of antibody production in a simple and easy manner. Said method is useful in hybridoma screening and/or hybridoma cloning.

### BACKGROUND OF THE INVENTION

Since the success of G. Köler and C. Milstein in forming hybridomas capable of monoclonal antibody production by fusion between mouse-derived splenocytes and myeloma cells [Nature, 256, 495 1975)], a large number of monoclonal antibodies have been produced and used as reagents for research purposes, diagnostic reagents or therapeutic agents.

While various modifications have been devised to meet respective requirements, monoclonal antibodies are produced most generally by the following steps.

(1) Lymphocytes of an immunized animals (e.g. mouse) are fused with a permanent or immortal cell line (e.g. myeloma cells) using a fusion promoter such as polyethylene glycol or by electric stimulation. (2) The resulting cell population composed of lymphocytes and myeloma cells submitted to fusion but remaining unfused and hybridomas formed by the fusion procedure is sowed into wells of microwell plates so as to give a density of about one fused cell/well. (3) Hybridomas alone were caused to proliferate by incubating in a selective medium (e.g. HAT medium). (4) The hybridoma culture supernatants are sampled and assayed for antibody titer by ELISA or the like method. (5) For the cells in antibody-positive wells, cloning and antibody screening are repeated to give cell lines capable of monoclonal antibody production.

However, the prior art method mentioned above is complicated and required much labor for medium exchange, microscopic observation of the state of proliferation, and antibody productivity assay, among others. Thus, in the early incubation period until the appearance of hybridoma clones, medium exchange must be done in more than several hundred microwells at 3- or 4-day intervals. Furthermore, since when cell proliferation reaches a stage such that cells cover the whole culture surface of microwells, cell deaths increase abruptly, it is necessary to observe the state of cell proliferation, collect culture supernatants when necessary, and quickly perform antibody titer assays.

In addition, since the rate of hybridoma proliferation varies from well to well, cell observation, culture supernatant collection and culture supernatant antibody titer assaying must be made almost ever day during that period.

### SUMMARY OF THE INVENTION

Accordingly, it is an object of the invention to provide a novel method of distinguishing cells to thereby markedly reduce the labor required for cell observation and medium exchange.

To solve the above problems, the invention uses, for distinguishing cells, the technique comprising culturing cells each enclosed in a semipermeable microcapsule. Thus the invention provides a method of distinguishing hybridoma cells capable of producing a monoclonal antibody from cells incapable of antibody production which comprises:
(A) culturing, in a selective medium, the cell fusion mixture derived from lymphocytes and a permanent cell line to thereby cause selective proliferation of hybridomas formed by the fusion procedure,
(B) enclosing the cell population in a large number of microcapsules and culturing the same in that microencapsulated state to thereby confine the antibodies produced by hybridomas in the respective capsules, and
(C) binding a labeled antibody-binding substance to the antibodies in the capsules to thereby detect the presence or absence of an antibody in each capsule and thus distinguishing antibody-producing hybridoma cells from cells incapable of antibody production.

### DETAILED DESCRIPTION OF THE INVENTION

As the microcapsule-forming material, there may be mentioned, for example, sodium alginate, carrageenin and pectin. Said material is not limited to these but any water-soluble acidic polysaccharide gum may also be used.

Microencapsulation of cells can be effected, for example, by merely adding an acidic polysaccharide gum solution mixed with cells dropwise to an aqueous solution of a polyvalent cation. A CaCl₂ solution, for instance, may be used as the aqueous solution of polyvalent cation, and the concentration thereof is not critical. It is sufficient if said solution contains the calcium ion in an amount sufficient to crosslink the polysaccharide molecules. The means of dropping is, for example, a syringe-type injector or a separatory funnel but is not limited to these. The rate of dropping may be accelerated by creating an air stream at the site of dropping from the dropping means.

The size, or diameter, of microcapsules can be controlled by means of the rate of dropping of the polysaccharide gum solution into the polyvalent cation-containing aqueous solution. Thus, for example, when the nozzle of the dropping means has a bore diameter of 0.5 mm, the air stream along the nozzle has a rate of flow of 6 liters per minute, and the rate of dropping is within the range of 0.1 ml per minute to 0.5 ml per minute, a capsule size adequate for encapsulating at most one hybridoma in one microcapsule can be obtained.

The microcapsule wall or membrane must be such that substances necessary for cell growth can permeate it and the antibody produced by a cell can be retained in the microcapsule. For cell growth, it is sufficient, as experience teaches, that proteins with a molecular weight of about 40,000 can permeate said membrane. The capsule membrane has to meet, in addition to this requirement, the requirement that an antibody with a molecular weight of 150,000 should be retained in the capsule. Therefore, the capsule permeability is controlled by crosslinking the capsule surface layer using an appropriate crosslinking polymer. As the crosslinking polymer, there may be mentioned, for instance, polylysine, polypeptides and polyethylenimine. Among these, poly-L-lysine is particularly preferred. The cutoff molecular weight can be controlled by varying the concentration of the crosslinking polymer and the reaction time. Thus, for example, when poly-L-lysine with a molecular weight of 15,000 to 30,000, a membrane having the permeability mentioned above can be obtained by immersing microcapsules in a 0.05% solution of poly-L-lysine in physiological saline for 3 minutes.

Hybridomas enclosed in such capsules can proliferate, retaining their ability to produce and secrete antibodies. When cultured in a sufficient amount of medium, cells can be maintained at high density for a period of 4 weeks or longer. Therefore, when the cell population obtained by the fusion procedure is subjected to encapsulation and a large number'of the resulting cells are cultured in a culture bottle, the appearance of dead cells as resulting from excessive proliferation can be avoided and the labor needed for cell observation and medium exchange can be markedly reduced.

The culture bottle may be an ordinary polystyrene bottle or a high-density cell culture apparatus (e.g. of the Shimadzu SHC series), for instance. While the culture fluid comprises a culture medium containing all components necessary for continuous survival, metabolism and mitosis, such medium need not be encapsulate together with cells.

The technique of enclosing cells in semipermeable microcapsules and culturing the same is already known, as can be seen in Japanese Patent Publication No. 62-4919, for instance. In the above publication, however, it is an object to produce a substance producible by cells and, therefore, each capsule contains a number of cells of the same species. In addition, said cells are cells being stably cultured over a number of generations. Therefore, even when the environment in the capsule is not the best, cells can survive and, even if cells die with a considerable probability, remaining cells proliferate and the intended object can be achieved accordingly.

On the other hand, in the case of hybridoma screening in which an object of the invention lies, cells immediately after fusion are relatively weak and such weak cells must be microencapsulated, in principle, at a density of at most one fused cell per capsule. The object can be accomplished only when encapsulation conditions and culture conditions are optimal for cell proliferation.

The antibody produced by hybridomas multiplied in a capsule is prevented from secreting by the crosslinking polymer membrane on the capsule surface and is accumulated in the capsule. In this condition, a labeled antibody-binding substance capable of permeating said capsule is reacted with the capsule to thereby recognize the antibody stored in the capsule. The antibody-binding substance includes, among others, proteins such as protein A and protein G, antibodies reduced in molecular weight, and antigens. Protein A is particularly preferred, however.

The antibody-binding substance should have a molecular weight of not more than 100,000 so that it can permeate the capsule membrane.

Protein A is a protein occurring in the cell wall of Staphylococcus aureus and is known to bind to most mammalian antibodies. For use in the practice of the invention, protein A should preferably have a molecular weight of 8,000 to 42,000.

Labelling can be accomplished by any one of a variety of known procedures. The substance for labeling the antibody-binding substance may be a radioactive isotope, a fluorescent substance, an enzyme or the like. More specifically, FITC (fluorescein isothiocyanate), which is a fluorescent substance, is preferred.

After reaction of the labeled antibody-binding substance with the intracapsular antibodies, labeled capsules are distinguished from unlabeled capsules (i.e. capsules without antibody production therein). When FITC-protein A is used as the labeled antibody-binding substance, labeled capsules emit a fluorescence and therefore, fluorescence-emitting capsules can be distinguished from nonfluorescent capsules. This is not limitative of the means of distinguishment or selection but a cell sorter-like apparatus or a micromanipulation apparatus, for instance, can be used for ready selection of labeled capsules.

Once selected, the labeled capsules are disrupted, and the hybridomas are recovered. For disrupting the capsule membrane, it is necessary that the capsules be first taken out of the culture bottle and thoroughly washed so that all extracapsular contaminants can be removed.

The membrane can readily be disrupted in a conventional manner, for example by dispersing microcapsules in a solution containing calcium chloride and heparin, incubate the suspension and then adding the same to a sodium citrate solution. The concentrations of calcium chloride, heparin and sodium citrate should suitably be selected within the respective ranges in which the hybridomas within the capsules will not be adversely affected. The method of membrane disruption is not limited to the one mentioned above, however.

The hybridomas released by membrane disruption are placed in a culture bottle and then can be cloned in a conventional manner. Prior to cloning, capsule membrane fragments, antibodies produced and the like should preferably be separated from the hybridomas and discarded.

In accordance with the invention, by simply observing hybridoma surrounding microcapsules in search of a label, it is possible to judge as to whether a hybridoma is an antibody-producing hybridoma or not.

### EXAMPLE

### Immunization and cell fusion:

An emulsion (200 µl) prepared by mixing a human transferrin solution in Dulbecco's phosphate buffered saline (PBS) (200 µg/ml) with an equal volume of complete Freund's adjuvant was intraperitoneally injected into a 4-week-old Balb/c mouse three times at 2-week intervals. Two weeks later, a transferrin solution (1 mg/ml) was further injected into the caudal vein of the mouse. Three days thereafter, the spleen was excised, teased and suspended in 5 ml of 0.17 M ammonium chloride for 10 minutes to destroy the erythrocytes and thereby provide spleen cells.

Cell fusion was carried out by the method of Galfre et al. [Nature, 266, 550 (1977)] using 1.5 x 10⁸ spleen cells prepared in the above manner and 3 x 10⁷ P3X63Ag8U.1 mouse myeloma cells in the presence of 50% polyethylene glycol (average molecular weight 1,500; Boehringer Mannheim). Cells after fusion were suspended in 50 ml of HAT medium and cultured in a 5% CO₂ atmosphere at 37°C for 6 days.

### Encapsulation and cell culture:

The cells cultured were harvested by centrifugation and then resuspended in 0.5 ml of HAT medium, followed by addition of 1.5 ml of a 2% sodium alginate solution containing 0.85% sodium chloride. This cell suspension was forced through a nozzle (0.5 mm in bore diameter) at a rate of 0.1 ml per minute and dropped into a 1.5% calcium chloride solution while air was caused to flow along the nozzle at a rate of 6 liters per minute for capsule size control. The resultant suspension in 1.5% calcium chloride was stirred for 15 minutes for gelation. The microcapsules thus produced were washed twice with a 0.5% glucose solution. The thus-prepared microcapsules (about 600 µm in diameter) were immersed in a 5% glucose solution (20 ml) containing 0.05% poly-L-lysine for 10 minutes for coating the capsule surface with the macromolecule, and then washed with two 20-ml portions of a 5% glucose solution. The microcapsules were further treated with a 5% glucose solution (20 ml) containing 0.03% sodium alginate for 4 minutes and then washed twice with 5% glucose.

The capsules were treated with HAT medium (20 ml) containing 0.05 M sodium citrate for 1.5 minutes, washed with two 20-ml portions of HAT medium and then suspended in 50 ml of HAT medium. Incubation was carried out in a 5% CO₂ atmosphere at 37°C. Culture medium exchange was performed on day 7 after encapsulation and then at one-week intervals.

### Staining of capsules:

At three weeks after encapsulation, the capsules were collected in an Eppendorf tube, washed twice with PBS and then stained by rotational stirring in PBS (1 ml) containing 2.5 µg of FITC-labeled protein A (Cappel). After washing three times with 1 ml of PBS, they were subjected to observation under a fluorescence micro-scope.

With the capsule containing an antibody-producing cell, distinct intense fluorescence can be observed as compared with the capsule containing a cell incapable of antibody production. It is apparent that antibody-producing cells can be distinguished from non-producing cells based on the diference in fluorescence intensity therebetween.

When compared with the complicated prior art method comprising sampling of the culture supernatant and assaying same for antibody titer by ELISA, the method of the invention has an effect that antibody-producing cells can be distinguished by simply reacting the capsules with an antibody-binding substance. Therefore, monoclonal-producing cells can be distinguished in a short period of time and this step can be smoothly followed by the subsequent cloning step. Thus the work required for monoclonal antibody production can be much simplified and reduced.

## Claims

1. A method of distinguishing hybridoma cells capable of producing a monoclonal antibody from cells incapable of antibody production which comprises:
(A) culturing, in a selective medium, the cell fusion mixture derived from lymphocytes and a permanent cell line to thereby cause selective proliferation of hybridomas formed by the fusion procedure,
(B) enclosing the cell population in a large number of microcapsules and culturing the same in that microencapsulated state to thereby confine the antibodies produced by hybridomas in the respective capsules, and
(C) binding a labeled antibody-binding substance to the antibodies in the capsules to thereby detect the presence or absence of an antibody in each capsule and thus distinguishing antibody-producing hybridoma cells from cells incapable of antibody production.

2. The method of Claim 1, wherein the membrane of the microcapsule is penetrated by proteins with a molecular weight of less than about 40,000 and not penetrated by an antibody with a molecular weight of more than 50,000.

3. The method of Claim 1, wherein the microcapsule membrane is a crosslinking polymer composed of polylysine, polypeptides and polyethylenimine.

4. The method of Claim 1, wherein the antibody-binding substance includes protein A, protein G, antibodies reduced in molecular weight, and antigens.

5. A method of Claim 1, wherein the labeled antibody-binding substance is a radioactive isotope, a fluorescent substance or an enzyme.

6. A method of cloning an antibody-producing hybridoma which comprises cloning an antibody-producing hybridoma distinguished by the method of Claim 1.

7. Apparatus for distinguishing hybridoma cells capable of producing a monoclonal antibody from cells incapable of antibody production which comprises:
(A) means for culturing, in a selective medium, the cell fusion mixture derived from lymphocytes and a permanent cell line to thereby cause selective proliferation of hybridomas formed by the fusion procedure,
(B) means for enclosing the cell population in a large number of microcapsules and culturing the same in that microencapsulated state to thereby confine the antibodies produced by hybridomas in the respective capsules, and
(C) means for binding a labeled antibody-binding substance to the antibodies in the capsules to thereby detect the presence or absence of an antibody in each capsule and thus distinguishing antibody-producing hybridoma cells from cells incapable of antibody production.

8. Apparatus for cloning an antibody-producing hybridoma which comprises :
means for cloning an antibody-producing hybridoma distinguished by the method of Claim 1.
